(19) Europäisches Patentamt
European Patent Office
Office européen des brevets

(11) **EP 3 915 673 A1**

(12) **EUROPÄISCHE PATENTANMELDUNG**

(43) Veröffentlichungstag:
**01.12.2021 Patentblatt 2021/48**

(21) Anmeldenummer: 20176368.7

(22) Anmeldetag: **25.05.2020**

(51) Int Cl.:
**B01J 4/00** (2006.01) **B01J 19/26** (2006.01)
**B01F 5/02** (2006.01) **A61K 9/00** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **leon-nanodrugs GmbH**
**81679 München (DE)**

(72) Erfinder: **HORSTKOTTE, Elke**
**81245 München (DE)**

(74) Vertreter: **Held, Stephan**
**Meissner Bolte Patentanwälte**
**Rechtsanwälte Partnerschaft mbB**
**Widenmayerstraße 47**
**80538 München (DE)**

(54) **VERFAHREN ZUR ÜBERPRÜFUNG DER FUNKTIONSFÄHIGKEIT EINER ANLAGE ZUR HERSTELLUNG VON NANOPARTIKELN DURCH GEZIELTE FÄLLUNG AUS ÜBERSÄTTIGTEN LÖSUNGEN**

(57) Die Erfindung betrifft ein Verfahren zur Überprüfung einer Anlage zur Herstellung von Nanopartikeln. Die Erfindung betrifft ferner die Verwendung von Itraconazol zur Überprüfung einer Anlage zur Herstellung von Nanopartikeln.

EP 3 915 673 A1

**Beschreibung**

Die Erfindung betrifft ein Verfahren zur Überprüfung einer Anlage zur Herstellung von Nanopartikeln. Die Erfindung betrifft ferner die Verwendung von Itraconazol zur Überprüfung einer Anlage zur Herstellung von Nanopartikeln.

Als Nanopartikel werden Partikel definiert, die mit einer Partikelgröße kleiner als 1000 nm gezielt hergestellt und verarbeitet werden, um z. B. bessere Eigenschaften erreichen zu können.

Nanopartikel spielen bei der Entwicklung neuer Pharmazeutika eine bedeutende Rolle. Nanopartikel können aufgrund ihrer geringen Größe beispielsweise die Blut-Hirn-Schranke überwinden oder gezielt in Körper- oder Tumorzellen aufgenommen werden ("Drug-Targeting"). Unter bestimmten Umständen werden Nanopartikel vom Körper nicht als Fremdkörper erkannt und damit nicht vom Immunsystem eliminiert. Anwendungen von nanotechnologisch hergestellten Arzneimitteln sind in vielen Fachgebieten der Medizin möglich. Arzneiformen, die nanoskalige Wirkstoffe enthalten, können in vielen Bereichen der Medizin eingesetzt werden. So können damit Fortschritte in der Heilung und Diagnostik verschiedenster Erkrankungen erzielt werden: Es gibt bereits eine Reihe von Anwendungsgebieten, in denen Patente erteilt oder Medikamente auf dem Markt bzw. in der klinischen Erprobung sind: unter anderem bei malignen Tumoren, Infektionen, Stoffwechsel-Erkrankungen wie Diabetes, Autoimmun-Erkrankungen, Transplantat-Abstoßung und bei der Therapie von Entzündung und Schmerz (i. European Science Foundation, Nanomedicine - An ESF - European Medical Research Councils (EMRC) Forward Look report, Duncan, R. et al., 2004, European Science Foundation: Straßburg. p. 1-48.; ii. Couvreur, P. and C. Vauthier, Nanotechnology: intelligent design to treat complex disease, Pharm Res, 2006, 23(7): p. 1417-50; iii. Damge, C., C.P. Reis, and P. Maincent, Nanoparticle strategies for the oral delivery of insulin, Expert Opin Drug Deliv, 2008, 5(1): p. 45-68). Weiterhin können nanotechnologische Formulierungen in der Dermatologie eingesetzt werden (Schafer-Korting, M., W. Mehnert, and H.C. Korting, Lipid nanoparticles for improved topical application of drugs for skin diseases, Adv Drug Deliv Rev, 2007, 59(6): p. 427-43). Ein weiteres Anwendungsgebiet ist die Diagnostik in verschiedenen Bereichen der Medizin, insbesondere aber in der Onkologie. Nanotechnologische Arzneiformen können folgende Vorteile gegenüber herkömmlich hergestellten Arzneimitteln haben (Vauthier, C. and P. Couvreur, Developing nanoparticle drug carriers, Pharmaceutical technology europe, 2007(1): p. 35-42):

- Verbesserung der Löslichkeit und Bioverfügbarkeit des Wirkstoffes,
- Erhöhung der Aufnahme (Resorption) des Wirkstoffes, z.B. bei Arzneimitteln, die auf der Haut angewendet werden,
- Stabilisierung von neuartigen, aktiven Wirkstoffen (z. B. Peptide, Proteine),
- gezielter Transport von Wirkstoffen, z. B. zu Krebszellen ("Drug Targeting") oder in das ZNS (Schmerztherapie),
- kontrollierte Wirkstoffabgabe an den Körper über längere Zeit ("Controlled drug release"),
- Ermöglichung spezieller Diagnoseverfahren, z. B. in der Tumordiagnostik.

Nanopartikel lassen sich durch verschiedene Verfahren herstellen. Ein Verfahren besteht darin, Partikel durch gezielte Fällung aus übersättigten Lösungen zu generieren.

Die Herstellung von Nanopartikeln im MicroJetReactor (MJR) ist in allen Parametern leicht beherrschbar und lässt sich auch in größeren Produktionsmaßstäben effizient und kostengünstig umsetzen. In einer MJR-Anlage fördern Pumpen ein Lösungsmittel mit einem darin enthaltenen Wirkstoff und ein Antisolvens zu je einer Eintrittsdüse mit Durchmessern zwischen 50 und 1500 $\mu$m (oder kleiner als 50 $\mu$m, z.B. 1 $\mu$m). Dort baut sich ein hydrodynamischer Druck auf, der in Abhängigkeit von dem Durchmesser der Düse und der Pumprate 100 bar und mehr erreichen kann. Bei Durchtritt durch die Düse wird die Flüssigkeit im Inneren der Reaktorkammer auf Geschwindigkeiten von beispielsweise 30 bis 80 m/s beschleunigt. In der Reaktorkammer treffen die Flüssigkeiten in einem Kollisionspunkt aufeinander und durchmischen sich aufgrund der hohen kinetischen Energie intensiv. Der Wirkstoff fällt aufgrund der Verdünnung des Lösungsmittels und einer Übersättigung des Lösungsmittelgemisches als Partikel aus.

Die Größe der erzeugten Partikel steuert wesentlich deren Eigenschaften und ihr biologisches Verhalten und ist daher ein kritisches Qualitätsmerkmal. Die Partikelgröße wird durch die Prozessparameter der Herstellung gesteuert. Daher ist es wichtig, die korrekte Funktionsweise einer Anlage zur Herstellung zu überprüfen und sicherzustellen.

Das bisher verwendete Verfahren benutzt die Fällung von Hydroxypropylmethylcellulosephthalat (5 mg/ml HPMCP HP-50 gelöst in einem Gemisch aus 85 % Ethanol und 15 % Wasser) aus einem ethanolischen Strom (Solvens-Strom) durch Mischen mit einem Wasserstrom (Antisolvens-Strom). Dabei entstehen Nanopartikel der Substanz im Bereich von 170 nm bis 200 nm, die eine breite und multimodale Verteilung aufweisen. Die Größe der so erzeugten Partikel ist allerdings nicht abhängig von den gewählten Prozessparametern, insbesondere dem Mischvolumenverhältnis von ethanolischem Strom zu Wasserstrom.

Somit kann nicht in einem schnellen und einfach durchführbaren Fällungsverfahren experimentell geprüft werden, ob innerhalb der Anlage die Soll-Prozessparameter auch tatsächlich den Ist-Prozessparametern entsprechen. Wünschenswert wäre beispielsweise, wenn bei Einstellung eines Soll-Mischvolumenverhältnisses von Solvens-Strom zu Antisolvens-Strom durch einen Benutzer an einem Computerterminal in einem einfachen und schnellen Fällungsverfahren experimentell geprüft werden könnte, ob das Ist-Mischvolumenverhältnis von Solvens-Strom zu Antisolvens-Strom dem eingestellten Soll-Mischvolumenverhältnis entspricht.

Mit den bisher verwendeten Validierungssubstanzen wie z.B. Hydroxypropylmethylcellulosephthalat ist beispielsweise eine Überprüfung der Entsprechung von Ist-Mischvolumenverhältnis und Soll-Mischvolumenverhältnis nicht möglich, weil die Größe der erzeugten Partikel nicht abhängig von den gewählten Prozessparametern, insbesondere dem Mischvolumenverhältnis von Solvens-Strom zu Antisolvens-Strom, ist. Wünschenswert wäre ein Verfahren, mit dem überprüft werden kann, ob das Ist-Pumpvolumen der ersten Pumpe und das Ist-Pumpvolumen der zweiten Pumpe dem jeweiligen Soll-Pumpvolumen entspricht.

Es besteht also Bedarf an einem geeigneten und verbesserten Verfahren. Der Erfindung liegt daher die Aufgabe zugrunde, ein Verfahren zur Validierung zu schaffen, das zu hinreichend belastbaren Ergebnissen hinsichtlich der Funktionsfähigkeit einer Anlage zur Herstellung von Nanopartikeln durch gezielte Fällung aus übersättigten Lösungen führt.

Die Erfindung wird in einem ersten Aspekt gelöst durch ein Verfahren zur Überprüfung einer Anlage zur Herstellung von Nanopartikeln, wobei die Anlage eine erste Pumpe zur Erzeugung eines ersten Stromes, eine zweite Pumpe zur Erzeugung eines zweiten Stromes und eine Kammer zum Mischen des ersten Stromes und des zweiten Stromes umfasst,

wobei das Verfahren die Schritte umfasst:

a) Mischen des ersten Stromes und des zweiten Stromes, wobei der erste Strom durch die erste Pumpe und der zweite Strom durch die zweite Pumpe erzeugt wird und wobei durch Einstellen eines Soll-Pumpvolumens für die erste Pumpe und eines Soll-Pumpvolumens für die zweite Pumpe ein Soll-Volumenverhältnis von erstem zu zweitem Strom festgelegt wird,

wobei der erste Strom ein organisches Lösungsmittel und eine Referenzsubstanz enthält und der zweite Strom Wasser und einen Oberflächenmodifikator, wie beispielsweise ein Tensid, enthält,

wobei das Soll-Volumenverhältnis von erstem zu zweitem Strom einen Wert zwischen 1:3 und 1:20 einnimmt,

wobei durch das Mischen des ersten Stromes und des zweiten Stromes zumindest ein Teil der Referenzsubstanz und zumindest ein Teil des Tensides als Partikel ausgefällt wird (mit anderen Worten, durch das Mischen des ersten Stromes und des zweiten Stromes bilden zumindest ein Teil der Referenzsubstanz und zumindest ein Teil des Tensides Partikel; mit nochmals anderen Worten, durch das Mischen des ersten Stromes und des zweiten Stromes wird zumindest ein Teil der Referenzsubstanz als Partikel ausgefällt und zumindest ein Teil des Tensides adsorbieren an den Partikeln);

b) Bestimmen der Partikelgröße und optional der Größenverteilung der ausgefällten (mit anderen Worten, gebildeten) Partikel;

c) ein- oder mehrmaliges Wiederholen von Schritt a) und Schritt b), wobei das Soll-Volumenverhältnis bei jeder Wiederholung einen anderen Wert zwischen 1:3 und 1:20 einnimmt;

d) Vergleichen der Partikelgrößen und optional der Größenverteilungen der ausgefällten (mit anderen Worten, gebildeten) Partikel mit den Werten der Soll-Volumenverhältnisse,

wobei die Referenzsubstanz eine Substanz ist, die dadurch definiert ist, dass die Partikelgröße ausgefällter (mit anderen Worten, gebildeter) Partikel durch das Volumenverhältnis von erstem zu zweitem Strom eingestellt wird.

Das Verfahren umfasst einen Schritt d) des Vergleichens der Partikelgrößen und optional der Größenverteilungen der ausgefällten Partikel mit den Werten der Soll-Volumenverhältnisse. Hierbei wird insbesondere geprüft, ob eine Veränderung des Soll-Volumenverhältnisses von erstem zu zweitem Strom zu der erwarteten Veränderung der Partikelgröße (und gegebenenfalls Größenverteilung) führt. Dabei wird im Allgemeinen ein etwa linearer Trend erwartet. Wird dies bejaht, so ist dies eine Bestätigung, dass das Ist-Volumenverhältnis dem Soll-Volumenverhältnis entspricht. Im Allgemeinen sinkt die Partikelgröße bei unveränderter Größenverteilung, wenn beim Mischen das Volumen des ersten Stromes gegenüber dem Volumen des zweiten Stromes verringert wird. Bei den Anlagen sind häufig nur geringe Änderungen an dem Volumenverhältnis von erstem zu zweitem Strom nötig. Bei den meisten Substanzen wirkt sich eine kleine Änderung an dem Volumenverhältnis von erstem zu zweitem Strom nicht merklich aus. Allerdings haben die Erfinder entdeckt, dass bestimmte Substanzen unter bestimmten Umständen als Referenzsubstanzen eingesetzt werden können.

Die Referenzsubstanz ist demnach eine Substanz, die dadurch gekennzeichnet ist, dass die Partikelgröße ausgefällter (Nano-)Partikel durch Prozessparameter (insbesondere das Volumenverhältnis von erstem zu zweitem Strom) genau und reproduzierbar eingestellt wird und die ausgefällten Partikel eine geringe Größenverteilung aufweisen. Die Referenzsubstanz ist nicht oder nur in geringem Maße wasserlöslich. Bevorzugt ist die Referenzsubstanz ein (pharmazeutischer) Hilfs- oder Wirkstoff. In einer besonders bevorzugten Ausführungsform ist die Referenzsubstanz ein (pharmazeutischer) Wirkstoff, insbesondere Itraconazol. Diese Substanz hat sich als besonders geeignet erwiesen, da die Partikelgröße ausgefällter Partikel sehr exakt durch das Volumenverhältnis von erstem zu zweitem Strom eingestellt werden kann und die ausgefällten Partikel eine geringe Größenverteilung aufweisen.

Itraconazol (ITZ, $C_{35}H_{38}Cl_2N_8O_4$, MW 705.6 g/mol) ist ein Wirkstoff aus der Gruppe der Triazol-Antimykotika zur Behandlung von Pilzinfektionen mit verschiedenen Erreger. Es ist unter anderem gegen Hefen, Dermatophyten und Schimmelpilze wirksam. Die Effekte beruhen auf der Hemmung der Ergosterol-Biosynthese in den Pilzen. ITZ wurde bereits 1991 zugelassen, und die Substanz ist einfach und kostengünstig erhältlich. ITZ ist ein weißes Pulver, das in Wasser praktisch unlöslich ist und sich in organischen Lösemitteln wie Aceton, Dimethylformamid und Tetrahydrofuran löst.

Vorzugsweise ist die Anlage zur Herstellung von Nanopartikeln eine Anlage zur Herstellung von Nanopartikeln durch Fällung von Nanopartikeln aus übersättigten Lösungen. Eine solche Solvens/Antisolvens-Fällung ist zum Beispiel aus der EP 2 550 092 und der EP 2 395 978 B1 bekannt. Vorzugsweise ist die Anlage ein (Micro-)Jetreaktor oder vorzugsweise umfasst die Anlage einen (Micro-)Jetreaktor. Ein (Micro-)Jetreaktor ist zum Beispiel aus der EP 1 165 224 B1, der EP 1 165 224 A2 oder der WO 2018/234217 A1 bekannt. Bei einem Jetreaktor werden zwei Ströme vermischt bzw. kollidieren miteinander. Entsprechend kann die Anlage weitere Bauteile umfassen, wie sie beispielsweise in der EP 1 165 224 B1, der EP 1 165 224 A2 oder der WO 2018/234217 A1 beschrieben sind.

Nanopartikel sind Partikel mit einer Teilchengröße von 1 bis 1000 nm.

Der erste Strom und der zweite Strom sind Flüssigkeitsströme bzw. flüssige Ströme. Vorzugsweise weisen der erste Strom und der zweite Strom jeweils eine Geschwindigkeit von 10 bis 500 m/s, bevorzugt von 10 bis 200 m/s, auf. Hauptsächlich wird die Geschwindigkeit des ersten Stromes und des zweiten Stromes durch die jeweilige Pumprate sowie den Durchmesser der Eintrittsdüse des Jetreaktors bewirkt.

In einer Ausführungsform wird durch das Verfahren überprüft, ob das Ist-Pumpvolumen der ersten Pumpe und das Ist-Pumpvolumen der zweiten Pumpe dem jeweiligen Soll-Pumpvolumen entspricht. Aus dem Ist- bzw. Soll-Pumpvolumen der ersten Pumpe und dem Ist- bzw. Soll-Pumpvolumen der zweiten Pumpe ergibt sich ein Ist- bzw. Soll-Volumenverhältnis von erstem zu zweitem Strom während des Mischens der beiden Ströme. Mit anderen Worten ist das Verfahren vorzugsweise ein Verfahren zur Überprüfung, ob das Ist-Volumenverhältnis von erstem zu zweitem Strom dem Soll-Volumenverhältnis entspricht.

Vorzugsweise umfasst/ist das Mischen des ersten Stromes und des zweiten Stromes ein Kollidieren des ersten Stromes und des zweiten Stromes.

Das organische Lösungsmittel kann ein Lösungsmittel oder ein Lösungsmittelgemisch umfassen/sein. Bevorzugt sind wassermischbare Lösungsmittel. In einer bevorzugten Ausführungsform ist das organische Lösungsmittel ausgewählt aus Aceton, Ethanol, Eisessig, Ameisensäure, Dimethylformamid und Tetrahydrofuran sowie Mischungen davon oder aus einer Mischung von Salzsäure (z.B. 3 mol/L Salzsäure) und Ethanol. In einer besonders bevorzugten Ausführungsform ist das organische Lösungsmittel Aceton (insbesondere wenn die Referenzsubstanz Itraconazol ist).

In allen Ausführungsformen nimmt das Soll-Volumenverhältnis von erstem zu zweitem Strom vorzugsweise einen Wert zwischen 1:4 und 1:13, besonders bevorzugt zwischen 1:5 und 1:11, ein.

Vorzugsweise werden Schritt a) und Schritt b) mindestens zweimal wiederholt, so dass mindestens drei verschiedene Werte des Soll-Volumenverhältnisses zwischen 1:3 und 1:20 von erstem zu zweitem Strom verwendet werden. Vorzugsweise werden Schritt a) und Schritt b) mindestens dreimal wiederholt, so dass mindestens vier verschiedene Werte des Soll-Volumenverhältnisses zwischen 1:3 und 1:20 von erstem zu zweitem Strom verwendet werden. In einer bevorzugten Ausführungsform werden Schritt a) und Schritt b) achtmal wiederholt, so dass neun verschiedene Werte des Soll-Volumenverhältnisses zwischen 1:3 und 1:20 von erstem zu zweitem Strom verwendet werden. In anderen bevorzugten Ausführungsformen werden Schritt a) und Schritt b) zwei-, drei-, vier-, fünf-, sechs- oder siebenmal wiederholt und entsprechend drei, vier, fünf, sechs, sieben oder acht verschiedene Werte des Soll-Volumenverhältnisses zwischen 1:3 und 1:20 von erstem zu zweitem Strom verwendet werden.

Die Verben "umfassen" und "enthalten" und ihre Konjugationen umfassen das Verb "bestehen aus" und seine Konjugationen. Die Begriffe "ein" oder "eine" umfassen den Begriff "mindestens ein(e)". Ausführungsformen der Erfindung können in beliebiger Weise kombiniert werden, es sei denn, dies ist eindeutig nicht beabsichtigt.

Es hat sich als wichtig erwiesen, dass ein Oberflächenmodifikator, beispielsweise ein Tensid, eingesetzt wird, damit die Referenzsubstanz als Referenzsubstanz wirken kann und die Partikelgröße ausgefällter (Nano-)Partikel durch Prozessparameter (insbesondere das Volumenverhältnis von erstem zu zweitem Strom) genau und reproduzierbar mit geringer Größenverteilung einstellbar ist. In einer bevorzugten Ausführungsform ist das Tensid ein Polymer aus Naphthalinsulfonsäure und Formaldehyd (vor allem bei Verwendung von Itraconazol als Referenzsubstanz). Die Moleküle des Oberflächenmodifikators adsorbieren auf der Oberfläche der Nanopartikel bei deren Bildung und verhindern so Wachstum und Aggregation. Erfindungsgemäß ist daher z.B. die Verwendung eines oder mehrere Tenside (oberflächenaktive, amphiphile Substanzen) ausgewählt aus a) Pluronic F-127 (Matteucci, M. E., Hotze, M. A., Johnston, K. P., & Williams, R. O., 2006, Drug Nanoparticles by Antisolvent Precipitation: Mixing Energy versus Surfactant Stabilization, Langmuir, 22(21), 8951-8959), b) D-$\alpha$-tocopherol polyethylene glycol 1000 succinate (TPGS) (Van Eerdenbrugh, B., Vermant, J., Martens, J. A., Froyen, L., Humbeeck, J. Van, Van den Mooter, G., & Augustijns, P., 2010, Solubility increases associated with crystalline drug nanoparticles: methodologies and significance, Molecular Pharmaceutics, 7(5), 1858-1870), c) Polyethylen-glycol-dimethylether 500 (DMPEG) (McComiskey, K. P. M., Mugheirbi, N. A., Stapleton, J., & Tajber, L., 2018, In situ monitoring of nanoparticle formation: Antisolvent precipitation of azole antifungal drugs, International Journal of Pharmaceutics, 543(1-2), 201-213) und d) Tamol NN9401 (Polymer aus Naphthalinsulfonsäure und Formaldehyd). Erfindungsgemäß ist, dass der zweite Strom das Tensid enthält, vorzugsweise in 0,01 bis 1 Gew.-%, bevorzugt etwa 0,1-Gew.-%. Es kann nicht ausgeschlossen werden, dass äquivalente Ergebnisse erzielt werden, wenn der erste Strom das Tensid enthält oder der erste und zweite Strom ein Tensid enthält.

Bevorzugte Ausführungsformen sind auch in den Ansprüchen angegeben.

In einer bevorzugten Ausführungsform besteht der erste Strom aus dem organischen Lösungsmittel und der Referenzsubstanz und der zweite Strom aus Wasser und dem Tensid. Vorteilhaft ist die Verwendung einer möglichst geringen Zahl an Komponenten, um das Verfahren möglichst einfach zu halten, Kontaminationen zu vermeiden und Kosten zu sparen. Überraschenderweise hat es sich dennoch als wichtig erwiesen, einen Oberflächenmodifikator zu verwenden.

In einer bevorzugten Ausführungsform wird in Schritt a) und in Schritt b) die Partikelgröße und die Größenverteilung der ausgefällten Partikel bestimmt und werden in Schritt d) die Partikelgrößen und Größenverteilungen der ausgefällten Partikel in Abhängigkeit des Wertes des Soll-Volumenverhältnisses von erstem zu zweitem Strom verglichen. Die Aussagekraft des Verfahrens kann gesteigert werden, wenn die die Größenverteilung miteinbezogen wird.

In einer bevorzugten Ausführungsform kristallisiert die Referenzsubstanz während der Ausfällung aus dem organischen Lösungsmittel.

In einer bevorzugten Ausführungsform liegt die Partikelgröße ausgefällter Partikel bei jedem Volumenverhältnis von erstem zu zweitem Strom zwischen 1:3 und 1:20 zwischen 180 und 500 nm, bevorzugt zwischen 180 und 320 nm. Die Erfinder haben entdeckt, dass dies für Itraconazol gilt. Mit Itraconazol lässt sich die Partikelgrößenverteilung besonders fein einstellen.

In einer bevorzugten Ausführungsform umfasst die Anlage zum Zuführen des ersten Stromes und des zweiten Stromes in die Kammer jeweils mindestens eine Düse, wobei der Durchmesser der mindestens einen Düse des ersten Stromes und der Durchmesser der mindestens einen Düse des zweiten Stromes derart gestaltet ist, dass ein Mischen des ersten Stromes und des zweiten Stromes gewährleistet ist und wobei der erste Strom und der zweite Strom jeweils durch die mindestens eine Düse zum Mischen in die Kammer geführt werden.

In einer bevorzugten Ausführungsform ist der Durchmesser der mindestens einen Düse des ersten Stromes und der Durchmesser der mindestens einen Düse des zweiten Stromes derart bemessen, dass in dem ersten Strom und in dem zweiten Strom ähnliche hydrodynamische Drücke auftreten, wobei bevorzugt ist, dass das Verhältnis des hydrodynamischen Druckes in dem ersten Strom zu dem hydrodynamischen Druck in dem zweiten Strom zwischen 1:0,5 und 1:2 beträgt. In einer Ausführungsform ist der Durchmesser der mindestens einen Düse des zweiten Stromes um den Faktor 2 bis 5, bevorzugt etwa 3, größer als der Durchmesser der mindestens einen Düse des ersten Stromes.

In einer bevorzugten Ausführungsform beträgt der Durchmesser der mindestens einen Düse des ersten Stromes 50 bis 400 $\mu$m, bevorzugt etwa 100 $\mu$m, und der Durchmesser der mindestens einen Düse des zweiten Stromes beträgt 200 bis 1200 $\mu$m, bevorzugt etwa 300 $\mu$m.

In einem zweiten Aspekt betrifft die Erfindung die Verwendung von Itraconazol zur Überprüfung einer Anlage zur Herstellung von Nanopartikeln, optional unter Verwendung eines erfindungsgemäßen Verfahrens.

Die Beschreibungen zum ersten Aspekt und bevorzugten Ausführungsformen davon beziehen sich auch auf den zweiten Aspekt.

Die Partikelgröße ("z-average") und die Größenverteilung ("PDI") der ausgefällten Partikel wird vorzugsweise bei 25 °C durch dynamische Lichtstreuung (DLS) bestimmt, vorzugsweise mit einem Zetasizer Nanoseries von Malvern Panalytical Ltd., und nach ISO 13321:1996 und ISO 22412:2017 berechnet. Die Partikelgröße wird als "z-average" angegeben, die durch Cumulanten-Analyse der Autokorrelationsfunktion erhalten wird. Der Polydispersity Index ("PDI") gilt als Maß für die Breite der Verteilung und ist eine dimensionslose Größe zwischen 0 und 1. Je kleine diese Zahl, desto schmalbandiger ist die Verteilung. Werte unter 0,4 zeigen im Allgemeinen eine gewissermaßen homogene Partikelgrößenverteilung, während Werte größer 0,7 anzeigen, dass die Probe eine sehr inhomogene Größenverteilung aufweist.

Die Software ist vorzugsweise Zetasizer V7.13. Als Messparameter werden vorzugsweise eingestellt:

i) Probe
Messprinzip: DLS Partikelgrößenverteilung
Probenmaterial: Polystyrollatexstandard
Brechungsindex des Probenmateriales: 1,590 (Polystyrollatexstandard bei 25 °C)
Absorption des Probenmateriales: 0,010
Dispersionsmedium: Wasser
Viskosität des Dispersionsmediums: 0,8872 mPa*s (Viskosität von Wasser bei 25 °C)
Temperatur: 25 °C
Äquilibrierungszeit: 60 s

Messzelle (Typ): Einwegküvette
Länge des optischen Pfades der Messzelle: 10 mm
ii) Messung
Position der Messzelle: Automatisch
Messwinkel: 173 °, Rückstreuung (Backscattering)
Messdauer: Manuell, Anzahl der Läufe: 15, Laufdauer 10 s
Anzahl der Messzyklen: drei, ohne Verzögerung dazwischen
Messmodus: Intensitätsmodus
Verzögerungszeit für große Partikel: Nein
Attenuation: Automatische Auswahl
Bevorzugt wird das Gerät vor der Bestimmung mit einer Dispersion eines Partikelgrößenstandards (etwa 100 nm) des Geräteherstellers validiert.

Vorzugsweise werden die Partikelgrößen direkt (innerhalb von 30 Minuten) nach dem Ausfällen oder immer nach Ablauf einer festgelegten Zeit (beispielsweise 2 Minuten) bestimmt.

Nachfolgend wird die Erfindung anhand eines Beispieles erläutert.

Zunächst wurden folgende Lösungen bereitgestellt:

- Tamol NH 9401 0,1 Gew.-% in demineralisiertem Wasser,
- Itraconazol 0,2 Gew.-% in Aceton,
- demineralisiertes Wasser als Spüllösung und
- Aceton als Spüllösung

Zum Lösen des Itaconazols wird das Aceton bevorzugt auf 35 °C erwärmt. Später kann die Lösung bei Raumtemperatur (25 °C) verwendet werden.

Die MJR-Anlage umfasste eine erste Pumpe und eine erste Düse zur Zufuhr eines ersten Stromes in die Kammer und eine zweite Pumpe und eine zweite Düse zur Zufuhr eines zweiten Stromes in die Kammer. Die erste Pumpe und die erste Düse (100 $\mu$m Durchmesser) wurde zunächst mit Aceton mit 28 mL/min gespült und die zweite Pumpe und die zweite Düse (300 $\mu$m Durchmesser) wurde mit demineralisiertem Wasser mit einem Durchfluss von 150 mL/min gespült.

Anschließend wurde die erste Pumpe und die erste Düse (100 $\mu$m Durchmesser) mit 0,2 Gew.-% Itraconazol in Aceton mit 28 mL/min gespült und die zweite Pumpe und die zweite Düse (300 $\mu$m Durchmesser) wurde mit einer Lösung von 0,1 Gew.-% Tamol NH 9401 in demineralisiertem Wasser mit einem Durchfluss von 150 mL/min gespült.

In der Kammer der Mikrojetreaktor-Anlage wurden die zwei Ströme vermischt (miteinander kollidiert). Der erste Strom war die Lösung von 0,2 Gew.-% Itraconazol in Aceton, der zweite Strom war die Lösung von 0,1 Gew.-% Tamol NH 9401 in demineralisiertem Wasser. In der Kammer herrschte Raumtemperatur (25 °C). Der hydrodynamische Druck im ersten Strom betrug 10 bar, im zweiten Strom 8 bar. Die Flussraten lagen zunächst bei 28,3 mL/min (erster Strom) und 148,4 mL/min (zweiter Strom). Hieraus ergab sich ein Soll-Volumenverhältnis von ca. 1:5. Es wurde während der Versuche folgendermaßen sichergestellt, dass das Ist-Volumenverhältnis dem Soll-Volumenverhältnis entspricht: Die Anlage umfasste je eine Waage unter dem Vorratsgefäß für den ersten und zweiten Strom, um die Fließrate und das Mischvolumenverhältnis von erstem Strom und zweitem Strom gravimetrisch kontrollieren zu können. Der Verbrauch der 0,1 % Lösung von Tamol NH 9401 und der 0,2 % Lösung von Itraconazol in Aceton wurde von den Waagen abgelesen, um das Ist-Volumenverhältnis von erstem Strom zu zweitem Strom zu kontrollieren. Das gravimetrisch ermittelte Ist-Volumenverhältnis von erstem Strom zu zweitem Strom wurde folgendermaßen berechnen:

$$[\text{Masse verbrauchter Lösung (0,2\% Itraconazol in Aceton) in g} \times \rho \text{ dieser Lösung (= 0,792 g/mL)}]$$

$$:$$

$$[\text{Masse verbrauchter Lösung (0,1\% Tamol) in g} \times \rho \text{ dieser Lösung (= 1,000 g/mL)}]$$

Alternativ sind in die Anlage zwei Messgeräte eingebaut, die den Fluss der beiden Ströme kontinuierlich messen und das Messergebnis kontinuierlich anzeigen.

Somit konnte das Ist-Volumenverhältnis von erstem Strom zu zweitem Strom mit dem Soll-Volumenverhältnis verglichen sowie die Funktionstüchtigkeit der Pumpen während der Versuche gravimetrisch überprüft werden.

Dann wurde das erfindungsgemäße Verfahren entwickelt, mit dem das Ist-Volumenverhältnis von erstem Strom zu zweitem Strom mit dem Soll-Volumenverhältnis verglichen sowie die Funktionstüchtigkeit der Pumpen ganz genau überprüft werden kann. Besonders vorteilhaft ist, dass das Übereinstimmen des Ist-Volumenverhältnisses von erstem Strom zu zweitem Strom mit dem Soll-Volumenverhältnis sowie die Funktionstüchtigkeit der Pumpen direkt an einem Fällungsprodukt bestimmt werden können.

Um mit dem erfindungsgegenständlichen Verfahren zu überprüfen, ob das Ist-Volumenverhältnis von erstem Strom zu zweitem Strom dem Soll-Volumenverhältnis entspricht und die Pumpen funktionstüchtig sind, wurde ein Fällungsverfahren unter den gerade beschriebenen Bedingungen durchgeführt. In kurzen Zeitabständen wurden dabei die Flussraten gemäß Tabelle 1 verändert und für jede Flussrate eine Probe der erhaltenen Suspension genommen. In diesen Proben wurden jeweils die resultierenden hydrodynamischen Drücke gemessen, der Lösemittelgehalt der Mischung berechnet und 1,5 Minuten nach dem Aufsammeln der Mischung deren Partikelgröße und die Größenverteilung bestimmt:

Tabelle 1 (Experiment am 26.11.2019).

| ID | Flussrate Wasser | Flussrate Aceton | Druck Wasser | Druck Aceton | Volumenverhältnis | Aceton | Partikel-größe | PDI |
|---|---|---|---|---|---|---|---|---|
| | ml/min | ml/min | bar | bar | | Vol% | nm | |
| 15 | 187,5 | 11,8 | 14,7 | 7,5 | 15,89 | 5,9% | 182,7 | 0,064 |
| 14 | 182 | 16,3 | 14,2 | 8,7 | 11,17 | 8,2% | 199,3 | 0,065 |
| 9 | 178,9 | 16,2 | 13,6 | 8,3 | 11,04 | 8,3% | 197,9 | 0,004 |
| 8 | 177 | 20,5 | 12,8 | 9,8 | 8,63 | 10,4% | 208,8 | 0,034 |
| 13 | 179,5 | 21,7 | 13,1 | 11,6 | 8,27 | 10,8% | 221,2 | 0,04 |
| 7 | 177 | 22,5 | 13,2 | 11,9 | 7,87 | 11,3% | 215,7 | 0,11 |
| 6 | 174,5 | 23,4 | 13,2 | 13,9 | 7,46 | 11,8% | 230,7 | 0,031 |
| 11 | 172,8 | 25,4 | 12,4 | 17,8 | 6,80 | 12,8% | 252,4 | 0,038 |
| 12 | 175,3 | 25,9 | 12,5 | 16 | 6,77 | 12,9% | 251,6 | 0,059 |
| 5 | 169,4 | 25,1 | 12,2 | 12,9 | 6,75 | 12,9% | 238,4 | 0,035 |
| 4 | 168,4 | 27 | 11,7 | 14,5 | 6,24 | 13,8% | 249,3 | 0,032 |
| 17 | 170 | 27,8 | 12,4 | 16,6 | 6,12 | 14,1% | 256,7 | 0,097 |
| WDH 2 | 140,1 | 23,5 | 8,5 | 19,1 | 5,96 | 14,4% | 274,8 | 0,057 |
| 3 | 153,2 | 27,8 | 9,4 | 16,2 | 5,51 | 15,4% | 273,4 | 0,057 |
| 1 | 147 | 29 | 9,4 | 16,6 | 5,07 | 16,5% | 325,9 | 0,174 |
| 2 | 140,3 | 27,9 | 8,3 | 19,8 | 5,03 | 16,6% | 320,6 | 0,081 |

Der Versuch wurde an zwei weiteren Tagen wiederholt. Die Daten sind in Tabellen 2 und 3 zusammengefasst.

Tabelle 2 (Experiment am 04.12.2019).

| ID | Flussrate Wasser | Flussrate Aceton | Druck Wasser | Druck Aceton | Volumenverhältnis | Aceton | Partikel-größe | PDI |
|---|---|---|---|---|---|---|---|---|
| | ml/min | ml/min | bar | bar | | Vol% | nm | |
| 9 | 179,4 | 14,9 | 14,1 | 10,2 | 12,0 | 7,7% | 210,6 | 0,056 |
| 8 | 177,8 | 17,6 | 13,4 | 10,8 | 10,1 | 9,0% | 215,2 | 0,131 |
| 7 | 178 | 20,2 | 12,6 | 11,3 | 8,8 | 10,2% | 214,6 | 0,083 |
| 6 | 174 | 20,9 | 13,4 | 13,8 | 8,3 | 10,7% | 225,2 | 0,087 |
| 5 | 170,8 | 22,6 | 12 | 13,1 | 7,6 | 11,7% | 223,8 | 0,07 |
| 4 | 169,1 | 25,7 | 12,2 | 13,8 | 6,6 | 13,2% | 241,6 | 0,06 |

(fortgesetzt)

| ID | Flussrate Wasser | Flussrate Aceton | Druck Wasser | Druck Aceton | Volumenverhältnis | Aceton | Partikel-größe | PDI |
|---|---|---|---|---|---|---|---|---|
| | ml/min | ml/min | bar | bar | | Vol% | nm | |
| 3 | 155,2 | 25,5 | 9,3 | 14,4 | 6,1 | 14,1% | 253,7 | 0,077 |
| 1 | 148,7 | 25,6 | 9,4 | | 5,8 | 14,7% | 269,8 | 0,048 |
| 2 | 138,9 | 25,9 | 8,4 | 14,4 | 5,4 | 15,7% | 270,2 | 0,098 |

Tabelle 3 (Experiment am 23.10.2019).

| ID | Flussrate Wasser | Flussrate Aceton | Druck Wasser | Druck Aceton | Volumenverhältnis | Aceton | Partikel-größe | PDI |
|---|---|---|---|---|---|---|---|---|
| | ml/min | ml/min | bar | bar | | Vol% | nm | |
| 9 | 178,2 | 15,7 | 13,6 | 10,2 | 11,4 | 8,1% | 201,9 | 0,083 |
| 8 | 177 | 19,8 | 13,6 | 11,6 | 8,9 | 10,1% | 226,8 | 0,109 |
| 7 | 177,7 | 22,4 | 13 | 13,8 | 7,9 | 11,2% | 240,4 | 0,032 |
| 6 | 174,7 | 24,3 | 12,7 | 16,9 | 7,2 | 12,2% | 250,6 | 0,078 |
| 5 | 175,1 | 26 | 12,4 | 17,6 | 6,7 | 12,9% | 266,7 | 0,068 |
| 4 | 170,5 | 27,2 | 11,6 | 20,3 | 6,3 | 13,8% | 271,6 | 0,032 |
| 3 | 154,9 | 27,3 | 11,3 | 19,5 | 5,7 | 15,0% | 280,6 | 0,074 |
| 1WH | 148,5 | 29 | 12,5 | 21,9 | 5,1 | 16,3% | 299,7 | 0,07 |
| 2WH | 140,3 | 28,3 | 10,6 | 22,3 | 5,0 | 16,8% | 315,6 | 0,026 |

Der hydrodynamische Druck innerhalb des ersten und zweiten Stroms lag dabei in dem Bereich zwischen 6 und 23 bar. Die Bestimmung der Partikelgrößen und der Größenverteilung erfolgte wie im allgemeinen Teil beschrieben.

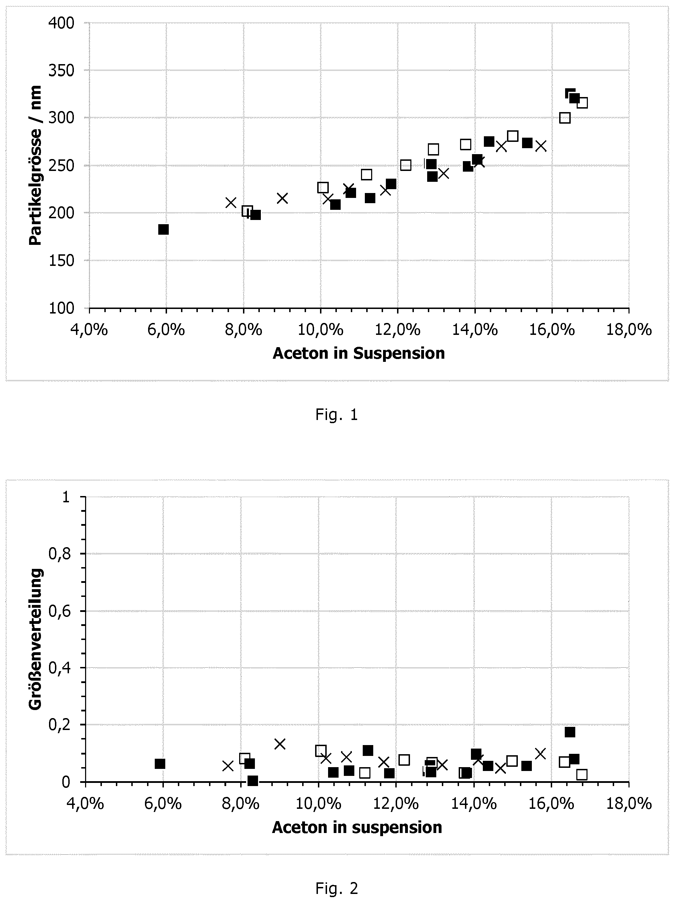

Die Ergebnisse sind graphisch in Fig. 1 und Fig. 2 dargestellt. Das Volumenverhältnis von erstem zu zweitem Strom ist auf der x-Achse als Konzentration (v/v) von Aceton dargestellt. Gravimetrisch wurde in den Versuchen sichergestellt, dass das Soll- dem Ist-Volumenverhältnis von erstem zu zweitem Strom entspricht. Die Fällung von Itraconazol aus Aceton gegen Wasser in Gegenwart von Tamol NH9401 führt zu qualitativ hochwerten Partikeln mit geringer Größenverteilung. Die Größe der Partikel kann über einen weiten Bereich zwischen 180 nm und 320 nm durch Variation eines Prozessparameters (Verhältnis der Flüssigkeitsströme) reproduzierbar eingestellt werden, wie Fig. 1 zeigt. Die Größenverteilung der Nanopartikel bleibt dabei gleich und gering, wie Fig. 2 zeigt. Andere Substanzen wie z.B. Hydroxypropylmethylcellulosephthalat fallen trotz unterschiedlicher Mischverhältnisse von Solvens und Antisolvens in nicht unterscheidbarer Partikelgröße aus.

Das erfindungsgemäße Verfahren liefert auf allen Anlagen unabhängig von deren Produktionskapazität reproduzierbar Nanopartikel identischer Größe mit geringer Größenverteilung. Wird keine Abweichung von den erwarteten Werte festgestellt, ist der Nachweis der Funktionsfähigkeit einer Anlage zur Herstellung von Nanopartikeln damit erbracht.

Bei einem Defekt einer der beiden Förderpumpen für die Flüssigkeiten zur Eintrittsdüse weicht die erzeugte Partikelgröße deutlich von erwarteten Wert ab und die Funktionsfähigkeit ist der Anlage ist nicht erbracht.

Somit stellt das erfindungsgemäße Verfahren ein schnelles und verlässliches Verfahren zur Überprüfung einer Anlage zur Herstellung von Nanopartikeln dar.

**Patentansprüche**

1. Verfahren zur Überprüfung einer Anlage zur Herstellung von Nanopartikeln,
   wobei die Anlage eine erste Pumpe zur Erzeugung eines ersten Stromes, eine zweite Pumpe zur Erzeugung eines zweiten Stromes und eine Kammer zum Mischen des ersten Stromes und des zweiten Stromes umfasst,
   wobei das Verfahren die Schritte umfasst:

a) Mischen des ersten Stromes und des zweiten Stromes, wobei der erste Strom durch die erste Pumpe und der zweite Strom durch die zweite Pumpe erzeugt wird und wobei durch Einstellen eines Soll-Pumpvolumens für die erste Pumpe und eines Soll-Pumpvolumens für die zweite Pumpe ein Soll-Volumenverhältnis von erstem zu zweitem Strom festgelegt wird,
wobei der erste Strom ein organisches Lösungsmittel und eine Referenzsubstanz enthält und der zweite Strom Wasser und ein Tensid enthält,
wobei das Soll-Volumenverhältnis von erstem zu zweitem Strom einen Wert zwischen 1:3 und 1:20 einnimmt,
wobei durch das Mischen des ersten Stromes und des zweiten Stromes zumindest ein Teil der Referenzsubstanz und zumindest ein Teil des Tensides als Partikel ausgefällt wird;
b) Bestimmen der Partikelgröße und optional der Größenverteilung der ausgefällten Partikel;
c) ein- oder mehrmaliges Wiederholen von Schritt a) und Schritt b), wobei das Soll-Volumenverhältnis bei jeder Wiederholung einen anderen Wert zwischen 1:3 und 1:20 einnimmt;
d) Vergleichen der Partikelgrößen und optional der Größenverteilungen der ausgefällten Partikel mit den Werten der Soll-Volumenverhältnisse,
wobei die Referenzsubstanz eine Substanz ist, die dadurch definiert ist, dass die Größe ausgefällter Partikel durch das Volumenverhältnis von erstem zu zweitem Strom eingestellt wird.

2. Verfahren zumindest nach Anspruch 1, **dadurch gekennzeichnet, dass** die Referenzsubstanz ein pharmazeutischer Wirkstoff, bevorzugt Itraconazol, ist.

3. Verfahren zumindest nach Anspruch 1 oder 2, **dadurch gekennzeichnet, dass** das organisches Lösungsmittel Aceton enthält, vorzugsweise Aceton ist.

4. Verfahren zumindest nach einem der vorherigen Ansprüche, **dadurch gekennzeichnet, dass** das Tensid ein Polymer aus Naphthalinsulfonsäure und Formaldehyd ist.

5. Verfahren zumindest nach einem der vorherigen Ansprüche, **dadurch gekennzeichnet, dass** der erste Strom aus dem organischen Lösungsmittel und der Referenzsubstanz besteht und der zweite Strom aus Wasser und dem Tensid besteht.

6. Verfahren zumindest nach einem der vorherigen Ansprüche, **dadurch gekennzeichnet, dass** in Schritt a) und in Schritt b) die Partikelgröße und die Größenverteilung der ausgefällten Partikel bestimmt wird und in Schritt d) die Partikelgrößen und Größenverteilungen der ausgefällten Partikel in Abhängigkeit des Wertes des Soll-Volumenverhältnisses von erstem zu zweitem Strom verglichen werden.

7. Verfahren zumindest nach einem der vorherigen Ansprüche, **dadurch gekennzeichnet, dass** die Referenzsubstanz während der Ausfällung aus dem organischen Lösungsmittel kristallisiert.

8. Verfahren zumindest nach einem der vorherigen Ansprüche, **dadurch gekennzeichnet, dass** die Partikelgröße ausgefällter Partikel bei jedem Volumenverhältnis von erstem zu zweitem Strom zwischen 1:3 und 1:20 zwischen 180 und 500 nm, bevorzugt zwischen 200 und 320 nm, liegt.

9. Verfahren zumindest nach einem der vorherigen Ansprüche, **dadurch gekennzeichnet, dass** die Anlage zum Zuführen des ersten Stromes und des zweiten Stromes in die Kammer jeweils mindestens eine Düse umfasst, wobei der Durchmesser der mindestens einen Düse des ersten Stromes und der Durchmesser der mindestens einen Düse des zweiten Stromes derart gestaltet ist, dass ein Mischen des ersten Stromes und des zweiten Stromes gewährleistet ist und wobei der erste Strom und der zweite Strom jeweils durch die mindestens eine Düse zum Mischen in die Kammer geführt werden.

10. Verfahren zumindest nach Anspruch 9, **dadurch gekennzeichnet, dass** der Durchmesser der mindestens einen Düse des ersten Stromes und der Durchmesser der mindestens einen Düse des zweiten Stromes derart bemessen ist, dass in dem ersten Strom und in dem zweiten Strom ähnliche hydrodynamische Drücke auftreten, wobei bevorzugt ist, dass das Verhältnis des hydrodynamischen Druckes in dem ersten Strom zu dem hydrodynamischen Druck in dem zweiten Strom zwischen 1:0,5 und 1:2 beträgt.

11. Verfahren zumindest nach Anspruch 9 oder 10, **dadurch gekennzeichnet, dass** der Durchmesser der mindestens einen Düse des ersten Stromes 50 bis 400 $\mu$m, bevorzugt etwa 100 $\mu$m, beträgt und der Durchmesser der mindestens einen Düse des zweiten Stromes 200 bis 1200 $\mu$m, bevorzugt etwa 300 $\mu$m, beträgt.

**12.** Verfahren zumindest nach einem der vorherigen Ansprüche, **dadurch gekennzeichnet, dass** das Verfahren ein Verfahren zur Überprüfung ist, ob ein Ist-Volumenverhältnis von erstem zu zweitem Strom dem Soll-Volumenverhältnis entspricht.

**13.** Verfahren zumindest nach einem der vorherigen Ansprüche, **dadurch gekennzeichnet, dass** das Verfahren ein Verfahren zur Überprüfung des Ist-Pumpvolumens der ersten Pumpe und des Ist-Pumpvolumens der zweiten Pumpe ist.

**14.** Verwendung von Itraconazol zur Überprüfung einer Anlage zur Herstellung von Nanopartikeln, optional unter Verwendung eines Verfahrens zumindest nach einem der Ansprüche 1 bis 13.

Fig. 1

Fig. 2

Europäisches
Patentamt

European
Patent Office

Office européen
des brevets

**EUROPÄISCHER RECHERCHENBERICHT**

Nummer der Anmeldung

EP 20 17 6368

| | **EINSCHLÄGIGE DOKUMENTE** | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| Y | IGOR V. ZHIGALTSEV ET AL: "Bottom-Up Design and Synthesis of Limit Size Lipid Nanoparticle Systems with Aqueous and Triglyceride Cores Using Millisecond Microfluidic Mixing", LANGMUIR, Bd. 28, Nr. 7, 21. Februar 2012 (2012-02-21), Seiten 3633-3640, XP055150435, ISSN: 0743-7463, DOI: 10.1021/la204833h * Zusammenfassung * * Seite 3635; Abbildung 2 * * Seite 3637, Absatz 3.5 * ----- | 1-14 | INV. B01J4/00 B01J19/26 B01F5/02 A61K9/00 |
| Y | APPALAKUTTI SUMANTH ET AL: "Process intensification of copper chromite (CuCr2O4) nanoparticle production using continuous flow microreactor", CHEMICAL ENGINEERING AND PROCESSING: PROCESS INTENSIFICATION, ELSEVIER SEQUOIA, LAUSANNE, CH, Bd. 89, 3. Januar 2015 (2015-01-03), Seiten 28-34, XP029200486, ISSN: 0255-2701, DOI: 10.1016/J.CEP.2014.12.012 * Zusammenfassung * * Seite 30, Absatz 3.1; Abbildung 1 * * Seite 32, Absatz 3.2; Abbildung 5 * * Seite 34, Absatz 4 * ----- -/-- | 1-14 | RECHERCHIERTE SACHGEBIETE (IPC) B01J B01F |

Der vorliegende Recherchenbericht wurde für alle Patentansprüche erstellt

| Recherchenort | Abschlußdatum der Recherche | Prüfer |
|---|---|---|
| München | 9. November 2020 | Thomasson, Philippe |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.........................................................................
& : Mitglied der gleichen Patentfamilie, übereinstimmendes Dokument

EPO FORM 1503 03.82 (P04C03)

Seite 1 von 3

Europäisches
Patentamt

European
Patent Office

Office européen
des brevets

# EUROPÄISCHER RECHERCHENBERICHT

**Nummer der Anmeldung**

EP 20 17 6368

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | BARATH PALANISAMY ET AL: "Continuous flow synthesis of ceria nanoparticles using static T-mixers", CHEMICAL ENGINEERING SCIENCE, OXFORD, GB, Bd. 78, 22. April 2012 (2012-04-22), Seiten 46-52, XP028500242, ISSN: 0009-2509, DOI: 10.1016/J.CES.2012.04.032 [gefunden am 2012-05-02] * Zusammenfassung * * Seite 47, Absatz 2; Abbildung 1 * * Seite 47, Absatz 3 - Seite 48, Absatz 5 * | 1-14 | |
| | ----- | | |
| Y | HAMDALLAH SHERIF I ET AL: "Microfluidics for pharmaceutical nanoparticle fabrication: The truth and the myth", INTERNATIONAL JOURNAL OF PHARMACEUTICS, ELSEVIER, NL, Bd. 584, 12. Mai 2020 (2020-05-12), XP086181209, ISSN: 0378-5173, DOI: 10.1016/J.IJPHARM.2020.119408 [gefunden am 2020-05-12] * Zusammenfassung * * Seite 12; Abbildung 14 * * Seite 13, Absatz 4.3.1 - Absatz 5 * | 1-14 | RECHERCHIERTE SACHGEBIETE (IPC) |
| | ----- | | |
| | -/-- | | |

Der vorliegende Recherchenbericht wurde für alle Patentansprüche erstellt

| Recherchenort | Abschlußdatum der Recherche | Prüfer |
|---|---|---|
| München | 9. November 2020 | Thomasson, Philippe |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes
    Dokument

EPO FORM 1503 03.82 (P04C03)

**Europäisches Patentamt**
**European Patent Office**
**Office européen des brevets**

# EUROPÄISCHER RECHERCHENBERICHT

**Nummer der Anmeldung**

EP 20 17 6368

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | LINCE F ET AL: "Strategies to control the particle size distribution of poly-@e-caprolactone nanoparticles for pharmaceutical applications", JOURNAL OF COLLOID AND INTERFACE SCIENCE, ACADEMIC PRESS,INC, US, Bd. 322, Nr. 2, 15. Juni 2008 (2008-06-15) , Seiten 505-515, XP022659370, ISSN: 0021-9797, DOI: 10.1016/J.JCIS.2008.03.033 [gefunden am 2008-03-21] * Zusammenfassung * * Seite 510 - Seite 511; Abbildungen 7, 8 * | 1-14 | |
| Y | WO 2014/152200 A1 (CUREPORT INC [US]) 25. September 2014 (2014-09-25) * Zusammenfassung * * Absatz [0070]; Abbildung 2 * * Absatz [0038]; Abbildung 4 * * Absatz [0040]; Abbildung 6 * * Absatz [0089] * | 1-14 | |
| Y | WO 2013/059922 A1 (UNIV BRITISH COLUMBIA [CA]) 2. Mai 2013 (2013-05-02) * Zusammenfassung * * Seite 1, Absatz 3 * * Seite 12 - Seite 14; Abbildung 1 * * Seite 15 - Seite 16 * * Seite 16; Abbildungen 2A-2C * * Seite 18; Tabelle 1 * * Anspruch 1 * | 1-14 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 9. November 2020 | Thomasson, Philippe |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 20 17 6368

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-11-2020

| Im Recherchenbericht angeführtes Patentdokument | Datum der Veröffentlichung | Mitglied(er) der Patentfamilie | Datum der Veröffentlichung |
|---|---|---|---|
| WO 2014152200 A1 | 25-09-2014 | AU 2014240013 A1 | 13-08-2015 |
| | | CA 2898047 A1 | 25-09-2014 |
| | | CN 105188685 A | 23-12-2015 |
| | | EP 2968193 A1 | 20-01-2016 |
| | | JP 6450742 B2 | 09-01-2019 |
| | | JP 2016517423 A | 16-06-2016 |
| | | KR 20150127580 A | 17-11-2015 |
| | | US 2014348900 A1 | 27-11-2014 |
| | | US 2018000735 A1 | 04-01-2018 |
| | | WO 2014152200 A1 | 25-09-2014 |
| WO 2013059922 A1 | 02-05-2013 | CA 2853316 A1 | 02-05-2013 |
| | | EP 2770980 A1 | 03-09-2014 |
| | | EP 3069785 A1 | 21-09-2016 |
| | | JP 2015502337 A | 22-01-2015 |
| | | JP 2017081954 A | 18-05-2017 |
| | | JP 2019116478 A | 18-07-2019 |
| | | JP 2020121986 A | 13-08-2020 |
| | | US 2014328759 A1 | 06-11-2014 |
| | | US 2016214103 A1 | 28-07-2016 |
| | | US 2018280970 A1 | 04-10-2018 |
| | | WO 2013059922 A1 | 02-05-2013 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2550092 A
- EP 2395978 B1
- EP 1165224 B1
- EP 1165224 A2
- WO 2018234217 A1

**In der Beschreibung aufgeführte Nicht-Patentliteratur**

- **DUNCAN, R. et al.** European Science Foundation, Nanomedicine - An ESF - European Medical Research Councils (EMRC) Forward Look report. *European Science Foundation: Straßburg,* 2004, 1-48
- **COUVREUR, P. ; C. VAUTHIER.** Nanotechnology: intelligent design to treat complex disease. *Pharm Res,* 2006, vol. 23 (7), 1417-50
- **DAMGE, C. ; C.P. REIS ; P. MAINCENT.** Nanoparticle strategies for the oral delivery of insulin. *Expert Opin Drug Deliv,* 2008, vol. 5 (1), 45-68
- **SCHAFER-KORTING, M. ; W. MEHNERT ; H.C. KORTING.** Lipid nanoparticles for improved topical application of drugs for skin diseases. *Adv Drug Deliv Rev,* 2007, vol. 59 (6), 427-43
- **VAUTHIER, C. ; P. COUVREUR.** Developing nanoparticle drug carriers. *Pharmaceutical technology europe,* 2007, vol. 1, 35-42
- **MATTEUCCI, M. E. ; HOTZE, M. A. ; JOHNSTON, K. P. ; WILLIAMS, R. O.** Drug Nanoparticles by Antisolvent Precipitation: Mixing Energy versus Surfactant Stabilization. *Langmuir,* 2006, vol. 22 (21), 8951-8959
- **VAN EERDENBRUGH, B. ; VERMANT, J. ; MARTENS, J. A. ; FROYEN, L. ; HUMBEECK, J. VAN ; VAN DEN MOOTER, G. ; AUGUSTIJNS, P.** Solubility increases associated with crystalline drug nanoparticles: methodologies and significance. *Molecular Pharmaceutics,* 2010, vol. 7 (5), 1858-1870
- **MCCOMISKEY, K. P. M. ; MUGHEIRBI, N. A. ; STAPLETON, J. ; TAJBER, L.** In situ monitoring of nanoparticle formation: Antisolvent precipitation of azole antifungal drugs. *International Journal of Pharmaceutics,* 2018, vol. 543 (1-2), 201-213